# EUROPEAN PATENT SPECIFICATION

(11) **EP 0 486 993 B1**
(45) Date of publication and mention of the grant of the patent: **24.05.1995**
(21) Application number: 91119646.7
(22) Date of filing: 18.11.1991
(51) Int. Cl.: B01J 23/42, B01J 37/06, B01J 23/62, B01J 23/89

(54) **Preparation of a zinc aluminate supported dehydrogenation catalyst**
Herstellung eines Dehydrierungskatalysators auf Zinkaluminat Basis
Préparation d'un catalyseur de déshydrogénation à base d'aluminate de zinc

(30) Priority: 19.11.1990 US 615518
(43) Date of publication of application: 27.05.1992
(73) Proprietor: PHILLIPS PETROLEUM COMPANY, Bartlesville Oklahoma 74004 (US)
(72) Inventor: Schubert, Paul Frederick, Campbell, CA 95008 (US); Kubicek, Donald Hubert, Bartlesville, OK 74006 (US); Kidd, Dennis Raymond, Dewey, OK 74029 (US)
(74) Representative: Dost, Wolfgang, Dr.rer.nat., Dipl.-Chem.

(56) References cited:
- EP-A- 0 191 925
- FR-A- 2 150 253
- FR-A- 2 202 146
- US-A- 4 006 074

## Description

This invention relates to dehydrogenation catalysts. In one aspect this invention relates to removing chloride from porous catalyst supports which have been impregnated with a solution of a platinum chloride compound. In another aspect, this invention relates to hydrocarbon conversion processes using the platinum catalyst prepared by the novel method.

Platinum catalysts supported on a variety of porous supports have been used in many processes, such as hydrogenation, dehydrogenation, cyclization and various other hydrocarbon conversion processes.

FR-A-2150253 discloses a process for preparing a supported catalyst for conversion of hydrocarbons, i.a. dehydrogenation, which catalyst is prepared by using alumina, stannic chloride and hexachloroplatinic acid. FR-A-2202146 is an extension of FR-A-2150253 and discloses a similar process wherein instead of tin compounds rhenium or germanium compounds are further used. None of these catalysts contains zinc aluminate.

In general, the supported platinum catalyst have been prepared by a variety of processes, such as coprecipitation with the support such as alumina, followed by washing, drying and calcining. Alternatively, the porous support is first formed in any of a variety of discrete forms or pellets, and the porous support is then impregnated with an aqueous solution of a platinum metal compound, such as chloroplatinic acid, and the resulting composite is dried and calcined. Other methods than aqueous impregnation have also been employed, such as the use of a nonaqueous impregnation media with various other compounds.

The prior art methods have provided highly effective catalysts. However, the catalyst preparation processes for the highly effective catalysts usually require process steps which are inherently time consuming and costly. In this regard, a particularly costly process step is the dechlorination of platinum catalyst prepared by impregnating a porous catalyst support with platinum contained in an aqueous solution of chloroplatinic acid. In this process the chloride content of the finished catalyst is in the range of 0.6-0.9 wt% of the support compared to 0.6 wt% of the support of platinum. Dechlorination of the platinum catalyst must therefore be carried out to prevent severe corrosion of downstream equipment.

In the past dechlorination of a supported platinum catalyst containing excessive amounts of chloride was carried out in a costly, high temperature steaming step which required an extended period of time. See US-A-3 649 566 which discloses a high temperature treatment with steam in order to remove as much as possible of the undesired chloride. For example, an effective catalyst dechlorination step required 33 hours of steaming at 1000°F to achieve less than 0.05 wt% chloride. Thus, it is highly desirable to find a supported platinum catalyst preparation process which lessens the costly, time consuming dechlorination step. We have discovered such a process in which impregnated chloride can be rapidly removed from the catalyst support while retaining essentially all of the impregnated platinum, thus substantially reducing the time and cost of catalyst preparation.

Accordingly, it is an object of this invention to provide an improved method for the manufacture of supported platinum catalyst.

Another object of this invention is to provide a catalyst with proper amounts of tin and platinum promoters while having a low chloride content.

Another object of this invention is to reduce the operating cost of preparing supported platinum catalyst by simplifying the dechlorination of a catalyst which is prepared by impregnating a catalyst support with a chloride containing solution.

Another object of this invention is to provide extruded catalyst supports of sufficient strength, but which are simpler and less expensive to manufacture than tableted catalyst supports.

Still another object of this invention is to prevent corrosion in a reactor containing the catalyst.

### Summary of the Invention

In accordance with this invention, we have discovered an improved process for producing a supported, platinum containing, dehydrogenation catalyst which can be easily dechlorinated after platinum and chloride have been added to the support from a solution containing a platinum chloride compound. Thus, according to the invention there is provided a process for preparing a supported dehydrogenation catalyst as defined in claim 1. The dechlorination is achieved by first drying the platinum chloride impregnated support, followed by calcining at a temperature sufficient to anchor the platinum to the support, and finally subjecting the impregnated, dried and calcined catalyst support to a washing step under conditions sufficient to substantially remove all chloride ions from the support while substantialy retaining all of the platinum catalyst deposited on the support, and thereby producing the desired dehydrogenation catalyst.

Further aspects and additional advantages of the present invention will be apparent from the following detailed description of the preferred embodiments of the invention and examples, as illustrated by the drawings in which:

### Brief Description of the Drawings

FIG. 1 is a simplified process flow scheme illustrating catalyst preparation according to this invention.

FIG. 2 is a graph illustrating hot water washing of catalyst impregnated with a platinum compound which contains chloride.

FIG. 3 is a graph illustrating propane conversion in a dehydrogenation process.

FIG. 4 is a graph illustrating propylene selectivity in a dehydrogenation process.

FIG. 5 is a graph illustrating methane selectivity in a dehydrogenation process.

FIG. 6 is a graph illustrating carbon oxides selectivity in a dehydrogenation process.

### Detailed Description of the Preferred Embodiments

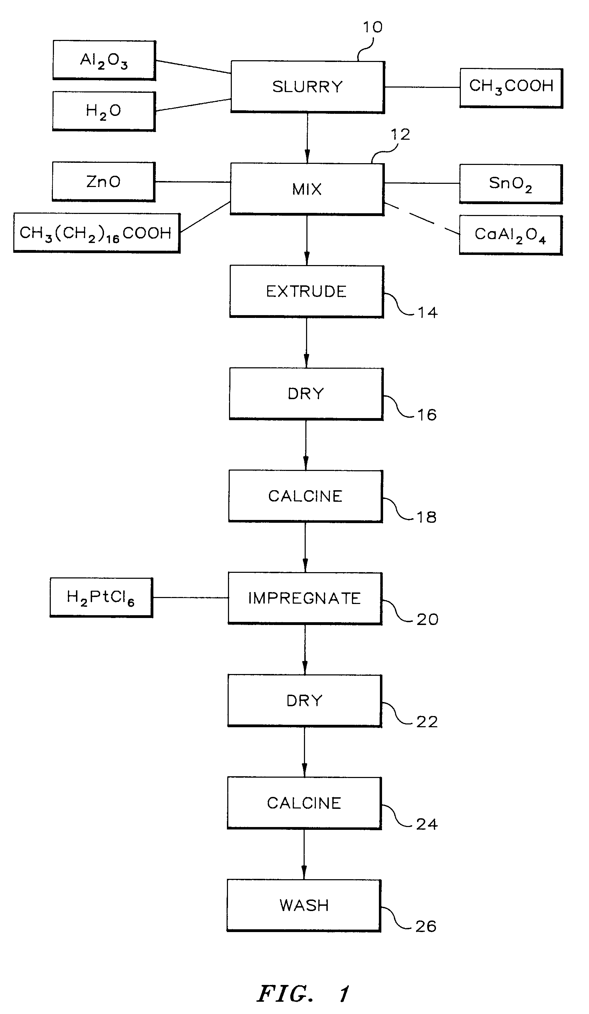

In a preferred embodiment, shown in drawing FIG. 1, a novel method for preparing supported platinum catalyst containing low levels of chloride is illustrated.

The catalyst composition, and the starting compounds used in the preparation of the catalyst composition, employed in this invention, are well known by those familiar in the art and are disclosed in numerous patents such as U.S. Patent No. 3,880,776 to Box, et al, and U.S. 3,668,151 to Walker.

The preferred catalysts of this invention are prepared by impregnating a porous support with an aqueous solution of a platinum metal compound. The platinum metal content of the catalyst is in the range of 0.01-5 weight percent of the support, and in preferred embodiments is in the range of 0.3-0.6 wt % of the support. Examples of simple platinum compounds that can be used for impregnation are: platinic chloride, chloroplatinic acid and ammonium chloroplatinate. Chloroplatinic acid, which is very effective, is preferred. When added to the support by impregnation from solution, chloroplatinic acid can be added from an aqueous or alcoholic solution. The porous support contains SnO/ZnAl₂O₄ (stannic oxide, zinc aluminate), and, optionally, calcium aluminate.

The catalyst support composition and the starting materials for the catalyst supports are also well known by those skilled in the art and are disclosed in numerous patents such as the aformentioned patents. Presently preferred is an extruded support comprising zinc aluminate and tin, in which tin exists as an oxide in the range of 0.01-5 weight percent of the support. The support may be prepared from mixtures of finely divided alumina, zinc oxide, and stannic oxide, and with the resulting composition extruded or tableted and then calcined at suitable temperatures. Additional constituents may be added to the support, if desired. Such additional constituents may include various lubricants, cements or other pelletizing additives. For example, calcium aluminate (for a cement) can exist in a range of 0-50 weight percent, and also stearic acid (octadecanoic acid), graphite or polyethylene fluff can be included for a lubricant in the range of 1-10 wt. %.

It is the the particular sequence of operations performed after the calcined extrudate has been impregnated with platinum and chloride, from a solution containing a platinum chloride compound, that provide the novel features of this invention.

Referring now to FIG. 1, there is illustrated the process flow for catalyst preparation according to this invention. The process begins with operations of a slurry preparation step 10, and a dry mixing step 12, which are employed to obtain a composite composition suitable for extruding.

In the slurry preparation step 10, an alumina slurry is obtained by mixing an alumina, glacial acetic acid and deionized water. In the mixing step 12, particulate zinc oxide, stannic oxide, stearic acid and optionally calcium aluminate are initially dry mixed in suitable proportions as in known in the art, then the slurry prepared in step 10 is slowly added to the dry mixture resulting in a composite composition which is suitable for extruding as illustrated in step 14 of FIG. 1. In some cases step 10 and step 12 can be combined.

The extrudate obtained in step 14 is dried in a first drying step 16 at a temperature of 109 to 172°C (228 °F to 342 °F), and the dried extrudate is passed to a first calcining step 18 at a temperature of 843°C (1550 °F) for a period of 5 hours. The dried and calcined catalyst support is then impregnated with platinum and chloride in any manner known in the art in step 20.

The presently preferred impregnation species for convenience and availability is H₂PtCl₆. Impregnation is conducted by treating the selected support with the aqueous solution of chloroplatinic acid at a temperature in the range of the freezing point to the boiling point of the solution, usually a temperature between 10 to 50°C (50-122°F) is suitable and convenient. Impregnation can be effected at any suitable pressure, generally atmospheric pressure is satisfactory. Time of impregnation can range such as 1 to 1000 seconds, or longer. Usually 5 to 100 seconds is suitable and convenient.

In accordance with the invention, the impregnated catalyst support, containing chloride, is passed in sequence to the second drying step 22; a second calcining step 24 at a temperature between 516 to 782°C (960-1440°F), and preferably between 582 to 643°C (1080 and 1190°F), which anchors the platinum to the support; and finally to a washing step 26 to remove the corrosive chloride without removing the platinum catalyst.

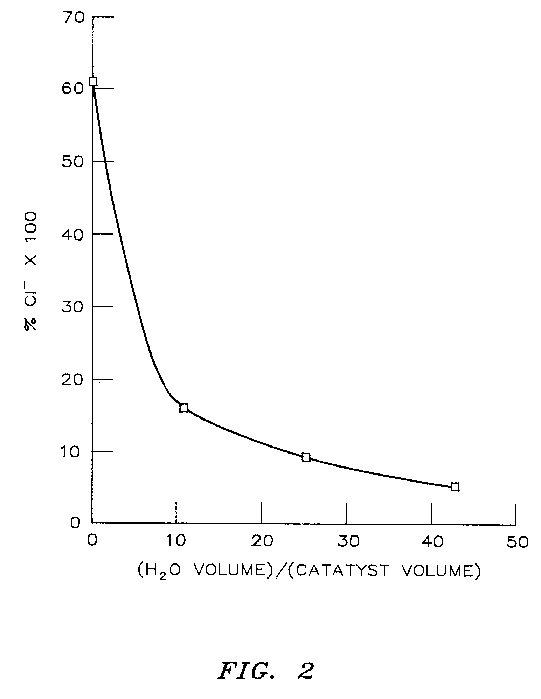

Referring now to FIG. 2, there is illustrated a plot of chloride content vs (water volume)/(catalyst volume) for washing the chloride containing catalyst with sufficient wash medium under conditions suitable to remove substantially all chloride ions from the catalyst. Suitable wash media includes water as well as organic solvents in which the chloride ions to be removed from the catalyst are soluble and/or form a complex with the chloride ions such as for example alcohols, esters, ethers and acids, as well as mixtures of any two or more of these media. Water, which is effective, is the preferred media.

The volume of wash media employed is not critical and can readily be determined by those of skill in the art. Generally, at least enough wash media should be employed to thoroughly contact the catalyst being treated. Preferably, sufficient quantity of wash media is employed to also provide a displacement wash, thus aiding in the removal of the chloride ions. In theory there is no upper limit as to the amount of wash media which can be employed. With reference to FIG. 2, however, preferably less than about 40 volumes of water/catalyst volume would be employed to minimize disposal and or recycle considerations. Preferably 10 to 30 volumes of wash media per volume of catalyst to be washed will be employed.

The conditions employed for the washing step are not critical and can be readily selected by those of skill in the art. While elevated as well as reduced temperatures can be employed, ambient conditions are preferred since good results are obtained at ambient conditions and minimum energy requirements are placed on the regeneration process when carried out under such conditions.

The supported platinum catalysts prepared according to this invention is used for dehydrogenation. These supported catalysts prepared in accordance with the present invention are particularly suitable for the dehydrogenation of alkanes containing from 2 to 12 carbon atoms per molecule. In one embodiment of this invention propane is dehydrogenated to propylene without producing large quantities of undesirable side products.

The following examples illustrate critical catalyst preparation steps and dechlorination steps according to this invention. These examples further illustrate propane dehydrogenation using the catalyst prepared according to this invention, and are intended to further assist one skilled in the art to an understanding of this invention. The examples given, reactants, and conditions are intended to be further exemplary, and not limitative of the reasonable scope of this invention.

### Example I

### Extruded Support

A zinc aluminate support containing stannic oxide and calcium aluminate was prepared by initially dry blending a mixture consisting of 15.6 g of finely divided reagent grade stannic oxide, 472.8 g of finely divided reagent grade zinc oxide, 128.4 g of Secar 71® (calcium aluminate cement) and 24 g of stearic acid (lubricant) for 10 minutes in a Lancaster Type PC mix-muller. A slurry obtained by mixing 604.5 g of flame-hydrolyzed alumina with 604 mL of a mixture comprising 102 mL of glacial acetic acid and 1 liter of deionized water was added to the stirred mixture of powders over a 15 minute period. The resulting paste was mixed an additional hour, then immediately extruded through a 0.32 cm (1/8 inch) die by means of a Baker-Perkins twin-screw extruder operating at 300 rpm. The extrudate having lengths ranging from 0.64 to 2.22 cm (1/4 inch to 7/8 inch) was collected and dried for 20 hours at 302°C (575°F) in a forced draft oven. The dried support was subsequently calcined in a muffle furnace for 5 hours at 843°C (1550°F). The calculated composition of the calcined support was 88.2 wt% zinc aluminate, 10.5 wt% calcium aluminate and 1.3 wt% stannic oxide. The average crush strength of the calcined extrudate was measured and found to be 5.45 kg (12 lbs). Based on mercury porosimetry results the pore volume was found to be 0.37 mL/g, the average pore radius was 29 nm (290 Angstroms) and the pore area was 25.3 m²/g.

### Example II

### Tabletted Support

A double cone blender was employed to mix 13.0 g of finely divided reagent grade stannic oxide, 394.0 g of finely divided reagent grade zinc oxide and 496.0 g of flame-hydrolyzed alumina for 15 minutes. The dry mix was stirred with sufficient deionized water (about 1 liter) to obtain a paste which was subsequently dried in an oven for 2 1/2 hours at 200°C (392°F). After cooling, the dried material was found and sieved to obtain particles of less than 0.6 mm (30 mesh) in size. The sieved fraction was tested and found to contain 5.15 wt% water. It was mixed with Secar 71® cement employing 10 g of the cement per 100 g of the sieved fraction calculated on a water-free basis. In this instance, 891.7 g of the ground material equivalent to 845.8 g of anhydrous material was mixed with 84.6 g of the cement and with 2 wt% of stearic acid as lubricant. This mixture was formed into 0.32 x 0.32 cm (1/8 x 1/8 inch) tablets by means of a Stokes BB2 tablet machine. The tablets were collected, steamed for 3 hours at 141°C (285°F) and calcined for 5 hours at 843°C (1550°F). The calculated composition of the calcined support was 89.6 wt% zinc aluminate, 9.1% calcium aluminate and 1.3 wt% stannic oxide. The average crush strength of the calcined tablets was 9.08 kg (20 lbs). Based on mercury porosimetry measurement the pore volume was found to be 0.30 mL/g, the average pore radius was 16.7 nm (167 Angstroms) and the pore area was 31 m²/g.

### Example III

### Catalyst Preparation and Dechlorination

Individual portions of the calcined, pelleted support of Example II were impregnated with aqueous chloroplatinic acid solution sufficient to obtain finished catalyst samples containing 0.3 wt% platinum and 0.6 wt% platinum based on the dry compositions. The chloride level impregnated in the catalyst corresponding to the 0.3 wt% loading of platinum was 0.57 wt% and to the 0.6 wt% platinum loading was determined to be 0.93 wt%. A portion of the chloride (about 0.1 wt%) resulted from that already present in the alumina. The samples were dried for 3 hours at 141°C (285°F).

The samples to be dechlorinated according to the invention were first calcined for 2 hours at 649°C (1200°F). Twenty-five gram portions of each calcined, cooled catalyst were washed with hot water for 6 hours in a soxhlet extractor, then removed and dried.

The control samples were first washed with hot water for 6 hours in the soxhlet extractor, then removed, dried and calcined for 2 hours at 649°C (1200°F).

The chloride levels resulting from each dechlorination method along with the corresponding platinum levels were determined. The results are presented in Table I.

**TABLE I**

| CATALYST DECHLORINATION | | | | | | |
|---|---|---|---|---|---|---|
| OPERATION | CONTROL | | INVENTION | | INVENTION | |
| | Pt. | Cl. | Pt. | Cl. | Pt. | Cl. |
| Impregnating | 0.6 | 0.9 | 0.3 | 0.6 | 0.6 | 0.9 |
| Calcine² | - | - | - | 0.6 | - | 0.7 |
| Washing | - | 0.06 | - | - | - | - |
| Calcine² | - | 0.06 | - | - | - | - |
| Washing¹ | - | - | - | 0.03 | - | 0.04 |
| Final | 0.4 | 0.06 | 0.3 | 0.03 | 0.6 | 0.04 |

| | | | | | | |
|---|---|---|---|---|---|---|
| (1) Washing consists of 6 hours in a SOXHLET extractor with water as a solvent. | | | | | | |
| (2) Calcining consists of 2 hours at 649°C (1200°F). | | | | | | |

The results illustrated in Table I clearly show that hot water washing effectively reduces the chloride content of both invention catalysts and control catalysts. However, the platinum level (0.6 wt% Pt) remains the same for the invention catalyst whereas the control catalyst lost 0.2 wt% Pt. The invention catalyst initially containing 0.3 wt% Pt retained its Pt level during the dechlorination process. Calcining the impregnated catalyst prior to dechlorination is therefore shown to be necessary to anchor platinum to the support.

The amount of water needed to decrease the chloride level of calcined catalyst containing 0.3 wt% Pt and 0.57% chloride to an acceptable level was determined with the soxhlet extractor containing 47 g of catalyst. The hot water needed was based on volumes of water per volume of catalyst. During the process, two 5 g samples were removed for chloride analysis. Although the sample removal altered the liquid to solids ratio somewhat it is believed that a good indication of the liquid volume needed for effective dechlorination was realized. The results are plotted in Figure 2. They show that the initial 0.57 wt% chloride level was reduced to about 0.17 wt% with the first 10 volumes of water, to about 0.09 wt% with about 25 volumes of water and to about 0.05 wt% with about 43 volumes of water. The chloride content decreases rapidly with the first few volumes of water and then it becomes increasingly difficult to reduce chloride to attain the desired level of 0.05 wt% or less.

### Example IV

### Propane Dehydrogenation

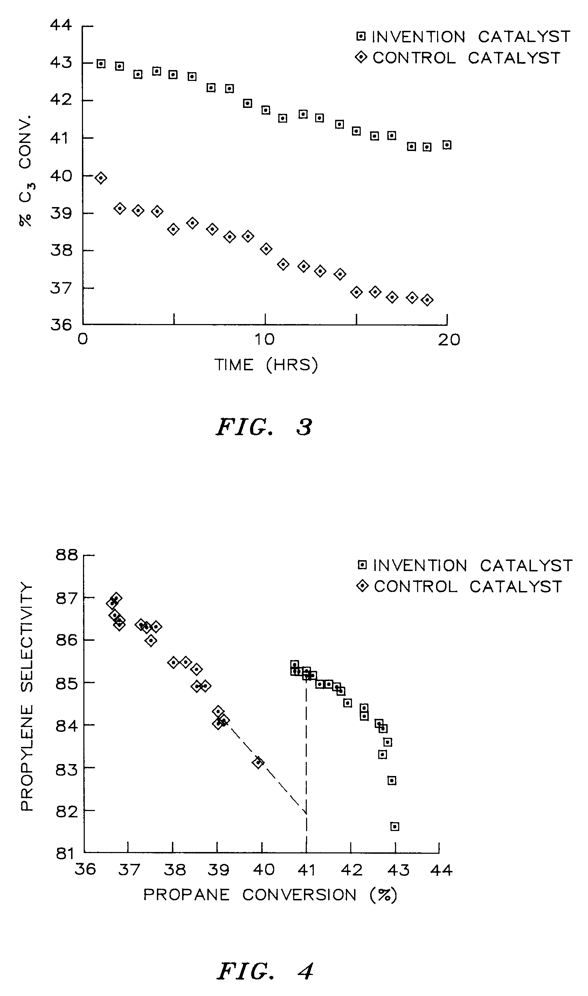

A portion of the calcined, hot water washed catalyst of Example III containing 0.3 wt% Pt and an identical calcined but unwashed control catalyst also containing 0.3 wt% Pt were steamed for 20 hours 649°C (1200°F) employing 16.8 liquid ml steam per minute, which is equivalent to 348 gas mL/minute at STP. A 5 g portion of each steamed catalyst was employed in propane dehydrogenation at 600°C (1112°F) in the substantial absence of oxygen using a laboratory catalytic reactor operating at 4 LHSV and 0.45 MPa (50 psig). A propane feed of 20 liquid mL/minute equivalent to 87 gas mL/minute at STP and steam of 16.8 liquid mL/minute were passed through the reactor. Each catalyst was broken in for 2 hours at test conditions with the feed and steam and then regenerated for 2 hours employing 100 mL air/minute and steam at 16.8 liquid mL/minute at test conditions. Each test cycle consisted of 20 hours on stream followed by 4 hours of regeneration. The performance of the catalysts was based on the second cycle test results and is shown in Figures 3, 4, 5 and 6. These results show the washed invention catalyst to be more active than the unwashed control catalyst.

The ability of the catalyst to convert propane to propylene without producing large quantities of undesirable side products is the desired feature. The data presented in Figure 3 show that the washed invention catalyst converts more propane to products than the unwashed control sample over the entire 20 hour test cycle. However, since the invention catalyst is always more active than the control catalyst, direct comparisons must come from interpolation of the selectivity data presented in Figures 4, 5, and 6. The data in Figure 4 indicate for a given propane conversion, for example 41%, the selectivity of the unwashed control catalyst to propylene would be approximately 82% based on the extension of the curve, while the washed invention catalyst is about 85.5%.

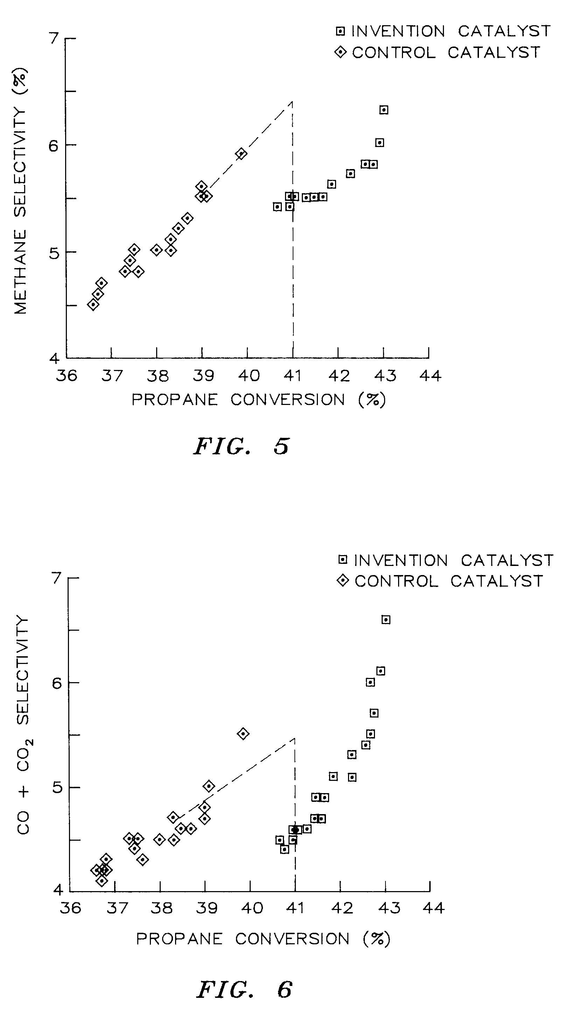

Figures 5 and 6 show the catalysts production of undesirable methane and carbon oxides, which have very low values in the propane conversion process since it is desirable to minimize their production. The data in Figure 5 show that at constant conversion, for example 41%, the selectivity to methane of the unwashed control catalyst would be approximately 6.4% based on the extension of the curve , while that of the washed invention catalyst is about 5.4%. The data in Figure 6 show that at constant conversion the washed invention catalyst will produce less carbon monoxide and carbon dioxide than the unwashed control catalyst. Thus, based on extension of the curve for the control catalyst to the 41% level as before, the unwashed control catalyst selectivity to carbon oxides would be approximately 5.5% compared to that of the washed invention catalyst is about 4.5%

The test results show that the invention catalyst exhibits better activity and selectivity in propane dehydrogenation than the control catalyst. Since both catalyst were steamed for 20 hours prior to testing the difference in results in believed to be a result of the hot water washing of the invention catalyst.

The above examples have been provided merely to illustrate the practice of our invention and should not be read so as to limit the scope of the appended claims in any way.

## Claims

1. A process for preparing a supported dehydrogenation catalyst, characterized by the following steps in the stated sequence:
(a) impregnating a porous catalyst support containing zinc aluminate, from 0.01 to 5 wt% stannic oxide, and, optionally, calcium aluminate, with a platinum and chloride containing solution to obtain a composite catalyst support containing platinum and chloride ions;
(b) drying said composite catalyst support;
(c) calcining the dried composite catalyst support of (b); and
(d) washing the composite catalyst support of (c) with hot water to obtain the desired dehydrogenation catalyst with the chloride ions removed and the platinum retained,
wherein the washed dehydrogenation catalyst of step (d) contains from 0.01 to 5 wt% platinum and less than 0.05 wt% chloride, the remainder being zinc aluminate, stannic oxide, and, optionally, calcium aluminate, if present.

2. The process of claim 1, wherein said impregnating step (a) is conducted at a temperature from 10 to 50°C for a time of 1 to 1000 seconds using an aqueous chloroplatinic acid solution having a platinum concentration to provide a platinum content of 0.1 to 5.0 wt% of said dehydrogenation catalyst.

3. The process of claim 1 or 2, wherein drying step (b) is conducted at a temperature of 109 to 172 °C and said calcining step (c) is conducted at a temperature of 516 to 782°C.

4. The process of claim 3, wherein drying step (b) is conducted at a temperature of 115 to 165°C and said calcining step (c) is conducted at a temperature of 549 to 749°C.

5. The process of any of the preceding claims being carried out to provide from 0.3 to 0.6 wt% platinum in said dehydrogenation catalyst.

6. The process of any of the preceding claims, wherein said porous support contains zinc aluminate, stannic oxide and calcium aluminate.

7. The process of claim 6, wherein said porous support contains about 89.6 wt% zinc aluminate, about 9.1 wt% calcium aluminate and about 1.3 wt% stannic oxide.

8. The process claim 6, wherein said porous support contains about 88.2 wt% zinc aluminate, about 10.5 wt% calcium aluminate and about 1.3 wt% stannic oxide.

9. The process of any of claims 1 - 8, wherein said porous support is an extruded support.

10. The process of any of claims 1 - 8, wherein said porous support is a tableted support.

11. The process of any of the preceding claims wherein the volume of hot water employed varies in the range of 10 - 43 volumes of hot water per volume of catalyst.

12. The process of claim 1, wherein said porous support used in step (a) has been obtained by
- blending a mixture of finely divided stannic oxide and finely divided zinc oxide, stearic acid, and optionally calcium aluminate cement to form a dry mixture;
- blending a mixture of alumina, deionized water and acetic acid to form a slurry of alumina;
- slowly adding said slurry of alumina to said dry mixture to form an extrudable composite composition;
- extruding said extrudable composite composition to form short strands of extrudate; and
- drying and calcining said extrudate to form said porous catalyst support.

13. The use of the dehydrogenation catalyst as obtained in any of claims 1- 12 in a process for dehydrogenating alkanes.

## Patentansprüche

1. Verfahren zur Herstellung eines trägergebundenen Dehydrierungskatalysators, gekennzeichnet durch die folgenden Stufen in der angegebenen Reihenfolge:
(a) Imprägnieren eines porösen Katalysatorträgers, der Zinkaluminat, 0,01 bis 5 Gew.-% Zinn(IV)-oxid und wahlweise Calciumaluminat enthält, mit einer Platin und Chlorid enthaltenden Lösung, um einen Mischkatalysatorträger, der Platin- und Chloridionen enthält, zu erhalten;
(b) Trocknen des Mischkatalysatorträgers;
(c) Calcinieren des getrockneten Mischkatalysatorträgers aus Stufe (b); und
(d) Waschen des Mischkatalysatorträgers aus Stufe (c) mit heißem Wasser, um den gewünschten Dehydrierungskatalysator zu erhalten, bei dem die Chloridionen entfernt sind und das Platin zurückgehalten wurde,
wobei der gewaschene Dehydrierungskatalysator aus Stufe (d) 0,01 bis 5 Gew.-% Platin und weniger als 0,05 Gew.-% Chlorid enthält, wobei es sich bei dem Rest um Zinkaluminat, Zinn(IV)-oxid und gegebenenfalls Calciumaluminat, falls dieses vorhanden ist, handelt.

2. Verfahren nach Anspruch 1, wobei die Imprägnierstufe (a) bei einer Temperatur von 10 bis 50°C für eine Zeitspanne von 1 bis 1000 Sekunden unter Verwendung einer wäßrigen Chloroplatin(IV)-säure-Lösung mit einer Platinkonzentration, um einen Platingehalt von 0,1 bis 5,0 Gew.-% des Dehydrierungskatalysators bereitzustellen, durchgeführt wird.

3. Verfahren nach Anspruch 1 und 2, wobei die Trocknungsstufe (b) bei einer Temperatur von 109 bis 172°C und die Calcinierungsstufe (c) bei einer Temperatur von 516 bis 782°C durchgeführt wird.

4. Verfahren nach Anspruch 3, wobei die Trocknungsstufe (b) bei einer Temperatur von 115 bis 165°C und die Calcinierungsstufe (c) bei einer Temperatur von 549 bis 749°C durchgeführt wird.

5. Verfahren nach einem der vorstehenden Ansprüche, das durchgeführt wird, um 0,3 bis 0,6 Gew.-% Platin in dem Dehydrierungskatalysator bereitzustellen.

6. Verfahren nach einem der vorstehenden Ansprüche, wobei der poröse Träger Zinkaluminat, Zinn(IV)-oxid und Calciumaluminat enthält.

7. Verfahren nach Anspruch 6, wobei der poröse Träger etwa 89,6 Gew.-% Zinkaluminat, etwa 9,1 Gew.-% Calciumaluminat und etwa 1,3 Gew.-% Zinn(IV)-oxid enthält.

8. Verfahren nach Anspruch 6, wobei der poröse Träger etwa 88,2 Gew.-% Zinkaluminat, etwa 10,5 Gew.-% Calciumaluminat und etwa 1,3 Gew.-% Zinn(IV)-oxid enthält.

9. Verfahren nach einem der Ansprüche 1-8, wobei es sich bei dem porösen Träger um einen extrudierten Träger handelt.

10. Verfahren nach einem der Ansprüche 1-8, wobei es sich bei dem porösen Träger um einen tablettierten Träger handelt.

11. Verfahren nach einem der vorstehenden Ansprüche, wobei das Volumen des eingesetzten heißen Wassers im Bereich von 10 bis 43 Volumina an heißem Wasser pro Volumen an Katalysator variiert.

12. Verfahren nach Anspruch 1, wobei der peröse Träger, der in Stufe (a) verwendet wird, durch
- Mischen eines Gemisches aus fein verteiltem Zinn(IV)-oxid und fein verteiltem Zinkoxid, Stearinsäure und wahlweise Calciumaluminat-Zement unter Bildung eines trockenen Gemisches;
- Mischen eines Gemisches aus Aluminiumoxid, entionisiertem Wasser und Essigsäure unter Bildung einer Aufschlämmung aus Aluminiumoxid;
- langsamer Zugabe der Aufschlämmung aus Aluminiumoxid zu dem trockenen Gemisch unter Bildung einer extrudierbaren Mischzusammensetzung;
- Extrudieren der extrudierbaren Mischzusammensetzung unter Bildung kurzer Stränge von Extrudat;
- Trocknen und Calcinieren des Extrudats unter Bildung des porösen Katalysatorträgers erhalten worden ist.

13. Verwendung des Dehydrierungskatalysators, der nach einem der Ansprüche 1-12 erhalten wurde, in einem Verfahren zur Dehydrierung von Alkanen.

## Revendications

1. Un processus pour préparer un catalyseur de déshydrogénation supporté, caractérisé par les étapes suivantes dans l'ordre indiqué :
(a) imprégnation d'un support poreux de catalyseur contenant de l'aluminate de zinc, de 0,01 à 5 % en poids d'oxyde stannique et, éventuellement, de l'aluminate de calcium, par une solution contenant du platine et un chlorure, de manière à obtenir un support composite de catalyseur contenant du platine et des ions chlorure ;
(b) séchage de ce support composite de catalyseur ;
(c) calcination du support composite séché de catalyseur de (b) ; et
(d) lavage du support composite de catalyseur de (c) à l'eau chaude pour obtenir le catalyseur de déshydrogénation souhaité avec les ions chlorure retirés et le platine conservé,
où le catalyseur de déshydrogénation lavé de l'étape (d) contient de 0,01 à 5 % en poids de platine et moins de 0,05 % en poids de chlorure, le reste étant de l'aluminate de zinc, de l'oxyde stannique et, éventuellement, de l'aluminate de calcium s'il est présent.

2. Le processus de la revendication 1, où l'étape (a) d'imprégnation est exécutée à une température de 10 à 50 °C pendant une durée de 1 à 1000 secondes en utilisant une solution aqueuse d'acide chloroplatinique ayant une concentration en platine permettant d'obtenir une teneur en platine de 0,1 à 5,0 % en poids du catalyseur de déshydrogénation.

3. Le processus de la revendication 1 ou 2, où l'étape (b) de séchage est exécutée à une température de 109 à 172 °C et l'étape (c) de calcination est exécutée à une température de 516 à 782 °C.

4. Le processus de la revendication 3, où l'étape (b) de séchage est exécutée à une température de 115 à 165 °C et l'étape (c) de calcination est exécutée à une température de 549 à 749 °C.

5. Le processus de l'une des revendications précédentes, mis en oeuvre pour obtenir de 0,3 à 0,6 % en poids de platine dans le catalyseur de déshydrogénation.

6. Le processus de l'une des revendications précédentes, où le support poreux contient de l'aluminate de zinc, de l'oxyde stannique et de l'aluminate de calcium.

7. Le processus de la revendication 6, où le support poreux contient environ 89,6 % en poids d'aluminate de zinc, environ 9,1 % en poids d'aluminate de calcium et environ 1,3 % en poids d'oxyde stannique.

8. Le processus de la revendication 6, où le support poreux contient environ 88,2 % en poids d'aluminate de zinc, environ 10,5 % en poids d'aluminate de calcium et environ 1,3 % en poids d'oxyde stannique.

9. Le processus de l'une des revendications 1 à 8, où le support poreux est un support extrudé.

10. Le processus de l'une des revendications 1 à 8, où le support poreux est un support pastillé.

11. Le processus de l'une des revendications précédentes, où le volume d'eau chaude employé varie dans la plage allant de 10 à 43 volumes d'eau chaude par volume de catalyseur.

12. Le processus de la revendication 1, où le support poreux utilisé à l'étape (a) a été obtenu par :
- malaxage d'un mélange d'oxyde stannique finement divisé et d'oxyde de zinc finement divisé, d'acide stéarique et éventuellement de cément d'aluminate de calcium de manière à former un mélange sec ;
- malaxage d'un mélange d'alumine, d'eau désionisée et d'acide acétique de manière à former une bouillie d'alumine ;
- addition lente de cette bouillie d'alumine à ce mélange sec de manière à former une composition composite extrudable ;
- extrusion de cette composition composite extrudable de manière à former des brins courts d'extrudat ; et
- séchage et calcination de cet extrudat de manière à former le support poreux de catalyseur.

13. L'utilisation du catalyseur de déshydrogénation obtenu par l'une des revendications 1 à 12 dans un processus pour déshydrogéner les alcanes.
